# EUROPEAN PATENT APPLICATION

(11) **EP 1 493 386 A1**
(43) Date of publication of application: **05.01.2005**
(21) Application number: 04014649.0
(22) Date of filing: 23.06.2004
(51) Int. Cl.: A61B 5/15

(54) **Lancing devices and methods of using the same**

(30) Priority: 30.06.2003 US 483409 P
(71) Applicant: Bayer HealthCare, LLC, Tarrytown, New York 10591 (US)
(72) Inventor: Kuriger, Rex, J., Granger, Indiana 46530 (US)
(74) Representative: Linhart, Angela, Dr.

(57) **Abstract**

A lancing device for drawing a body fluid sample from the skin that comprises a body portion, an endcap, a lancet and a vibration member. The endcap detachably connects to the body portion. The lancet is located within the body portion in a retracted position. The lancet is moveable between a retracted position and an extended position, and adapted to draw body fluid sample from the skin at a puncture site in an extended position. The vibration member extends through the endcap and is adapted to mechanically vibrate the skin adjacent to the puncture site. The lancet is adapted to draw the body fluid sample from the skin at a puncture site by extending through an opening formed in the endcap, the vibration member or the combination thereof. One example of a vibration member is a piezoelectric member.

## Description

### FIELD OF THE INVENTION

The present invention relates to lancing devices and methods of using the same, and more specifically, lancing devices for drawing a body fluid sample and methods of using the same that reduce or eliminate the pain to a test subject.

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, determining glucose in body fluids is important to diabetic individuals who must frequently check the glucose level in their body fluids to regulate the glucose intake in their diets.

One method of obtaining a body fluid sample such a whole blood sample is to use a lancing device. The whole blood sample may be used to monitor the glucose of an individual. Existing lancing devices pierce the tissue of the skin, allowing a blood sample to form on the skin's surface. The whole blood sample is then transferred to the testing device. The whole blood sample is often taken from the fingertips of a test subject for glucose monitoring because of the high concentration of capillaries that can provide an effective blood supply. Taking the blood from the fingertips, however, is disadvantageous because of the high concentration of nerve endings that cause pain and discomfort to many individuals. Other less painful testing sites such as forearms or thighs may be used in an attempt to avoid the sensitive fingertip region, but these sites generally provide a smaller blood sample.

It would be desirable to have a lancing device and a method of using a lancing device that would reduce or eliminate pain associated with taking of a body fluid sample.

### SUMMARY OF THE INVENTION

According to one embodiment, a lancing device for drawing a body fluid sample from the skin comprises a body portion, an endcap, a lancet and a vibration member. The endcap detachably connects to the body portion. The lancet is located within the body portion in a retracted position. The lancet is moveable between a retracted position and an extended position, and adapted to draw body fluid sample from the skin at a puncture site in an extended position. The vibration member extends through the endcap and is adapted to mechanically vibrate the skin adjacent to the puncture site. The lancet is adapted to draw the body fluid sample from the skin at a puncture site by extending through an opening formed in the endcap, the vibration member or the combination thereof.

According to another embodiment, a lancing device for drawing a body fluid sample from the skin comprises a body portion, an endcap, a lancet and a piezoelectric member. The endcap detachably c onnects t o he b ody p ortion. T he lancet is located within the body portion in a retracted position. The lancet is moveable between a retracted position and an extended position, and adapted to draw body fluid sample from the skin at a puncture site in an extended position. The piezoelectric member extends through the endcap and is adapted to mechanically vibrate the skin adjacent to the puncture site. The lancet is adapted to draw the body fluid sample from the skin at a puncture site by extending through an opening formed in the endcap, the piezoelectric member or the combination thereof.

According to one method, a lancing device for drawing a body fluid sample from the skin comprises providing a lancing device that comprises a body portion, an endcap, a lancet and a vibration member. The endcap is detachably connected to the body portion. The lancet is located within the body portion in a retracted position. The lancet is moveable between a retracted position and an extended position, and adapted to draw body fluid sample from the skin at a puncture site in an extended position. The vibration member extends through the endcap. The vibration member is placed in contact with the surface of the skin. The lancet is moved to an extended position such that a portion of the lancet protrudes through the opening formed in the endcap, the vibration member or the combination thereof. The vibration member is mechanically vibrated to the skin adjacent to a puncture site. A body fluid sample is taken from the skin via the lancet at the puncture site.

According to another method, a lancing device for drawing a body fluid sample from the skin comprises providing a lancing device that comprises a body portion, an endcap, a lancet and a piezoelectric member. The endcap is detachably connected to the body portion. The lancet is located within the body portion in a retracted position. The lancet is moveable between a retracted position and an extended position, and adapted to draw body fluid sample from the skin at a puncture site in an extended position. The piezoelectric member extends through the endcap. The piezoelectric member is placed in contact with the surface of the skin. The lancet is moved to an extended position such that a portion of the lancet protrudes through the opening formed in the endcap, the piezoelectric member or the combination thereof. The piezoelectric member is mechanically vibrated to the skin adjacent to a puncture site. A body fluid sample is taken from the skin via the lancet at the puncture site.

### BRIEF DESCRIPTION OF THE DRAWINGS

FIG. 1 is a perspective view of a lancing device according to one embodiment of the present invention;
FIG. 2 is an end view of the lancing device of FIG. 1 without the endcap and with the lancet being removed;
FIG. 3 is an end view of the lancing device of claim 1 without the endcap and including the lancet;
FIG. 4 is a perspective side view of the endcap depicted in FIG. 1;
FIG. 5 is a perspective side view of an endcap according to a further embodiment;
FIG. 6 is a perspective side view of an endcap according to a further embodiment; and
FIG. 7 is a perspective side view of an endcap according to a yet another embodiment.

While the invention is susceptible to various modifications and alternative forms, specific embodiments thereof have been shown by way of example in the drawing and will herein be described in detail. It should be understood, however, that it is not intended to limit the invention to the particular forms disclosed but, on the contrary, the intention is to cover all modifications, equivalents, and alternatives falling within the spirit and scope of the invention as defined by the appended claims.

### DETAILED DESCRIPTION OF THE ILLUSTRATED EMBODIMENT

The present invention is directed to lancing devices for drawing a body fluid sample from the skin and methods of using the same. The lancing devices of the present invention assist in reducing or eliminating pain associated with the drawing of the body fluid sample. The lancing device according to one embodiment comprises a body portion, an endcap, a lancet and a vibration member. According to one embodiment, the lancing devices of the present invention are adapted to draw a whole blood sample.

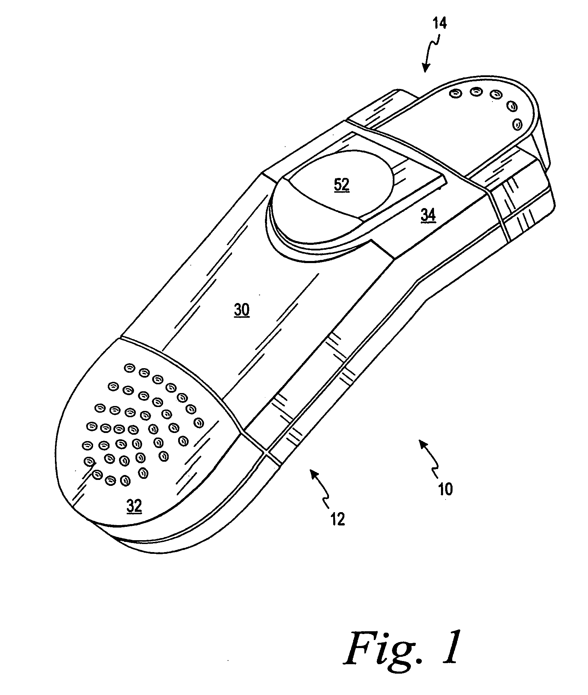

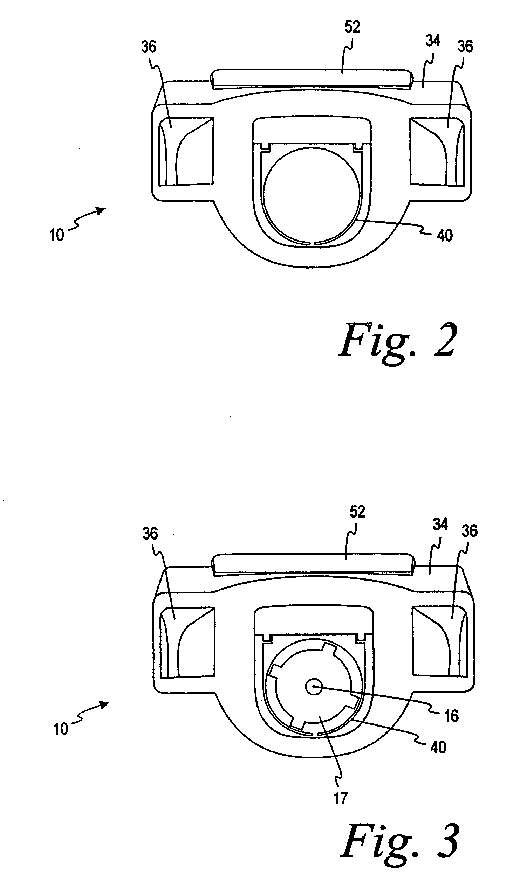

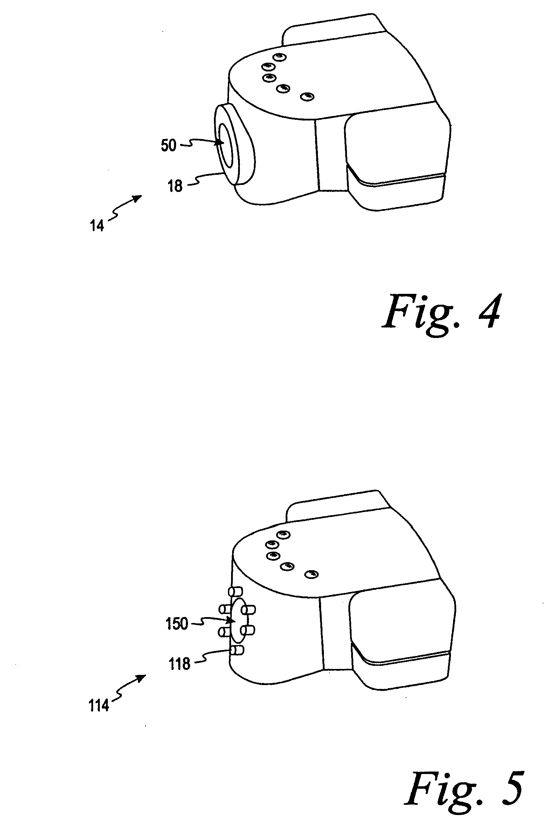

According to one embodiment, a lancing device 10 is depicted in FIG. 1. The lancing device 10 includes a body portion 12, an endcap 14, a lancet 16 (FIG. 3) and a vibration member 18 (FIG. 4). The body portion 12 includes a main body section 30, a moveable body section 32 movable relative to the main body section 30, and an endcap support section 34. To enable access to replace the lancet 16, the endcap 14 is detachably connected to the body portion 12. The endcap 14 is also desirably detachably connected to the body portion 12 because the endcap may contact blood and need to be discarded and replaced. Referring to FIG. 2, the endcap 14 may be detachably connected to the endcap support section 34 by a pair of latching or support arms 36, which is part of the endcap support section 34. It is contemplated that the endcap may be detachably connected to the body portion by other methods than shown in FIG. 2.

Referring to FIGs. 2 and 3, the lancet 16 of the lancing device 10 is mounted inside the body portion 12 via a lancet holder 40. As shown in FIG. 2, the lancet holder 40 is a device formed within the body portion that is adapted to hold and move the lancet 16. The lancet 16 may be encompassed by a base 17 such as shown in FIG. 3. The base 17 typically is a polymeric material, but may be formed of other materials.

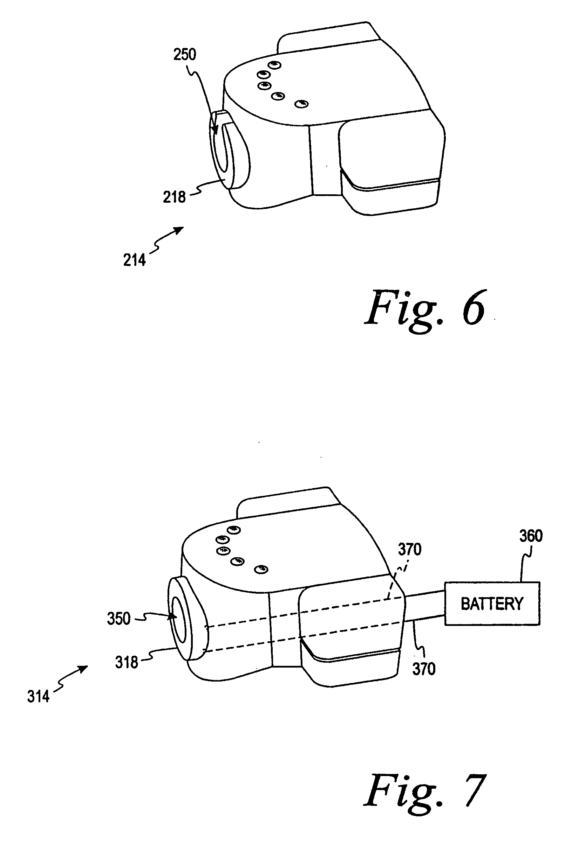

As shown in FIG. 4, for example, the lancet 16 is driven through an opening or aperture 50 formed in the vibration member 18 by a driving mechanism that is cocked by pulling the moveable body section 32 away from the body portion 12. It is contemplated that the lancet may be driven through an opening or aperture formed in the endcap (see FIG. 5). It is also contemplated that an opening may be formed in combination with the endcap and the vibration member in which the lancet is driven therethrough (see FIG. 6). Referring back to FIG. 1, t he d riving m echanism for d riving t he lancet 16 i fired b y pushing a button 52. The lancet 16 is spring-loaded, but it is contemplated in another embodiment that the lancet may not be spring-loaded. During the driving of the lancet, the tip of the lancet 16 according to one embodiment, momentarily protrudes through the opening 50. To vary the depth of the lancet 16 into the skin, a number of different sized endcaps may be provided according to another embodiment of the lancing device.

The lancet may be made of different materials. One material that may be used in forming the lancet 16 is stainless steel. It is contemplated that the lancet may be made of other metals or combinations thereof. The lancet may also be made of non-metals, such as polymers.

To reduce or eliminate the pain during the lancing process, the vibration member 18 is adapted to mechanically vibrate the skin adjacent to the puncture site. The vibration member 18 may be a ring such as shown in FIG. 4. It is contemplated that the vibration member may be other shapes such as a hollow square, rectangle, other polygonal shapes, oval, or other non-polygonal shapes, as well as a variety of other sizes. It is also contemplated that there may be multiple vibration members such as shown in FIG. 5 with endcap 114 and a plurality of vibrating members 118. The endcap 114 forms an opening 150 that allows the lancet to extend therethrough to draw the body fluid sample. Another example of a vibration member is shown in FIG. 6 with endcap 214 having a vibration member 218. The vibration member 218 is formed in a general U-shape and the opening 250 is formed in combination with the endcap 214 and the vibration member 218. The opening 250 allows the lancet to extend therethrough to draw the body fluid sample.

During the process of drawing the body fluid, the vibration member(s) contacts the skin. The mechanical vibration from the vibrating member(s) stimulates the nerves in the skin adjacent to the puncture site. This is believed to make the prick less noticeable, if noticeable at all to the test subjects. It is believed that this mechanical vibration assists in masking the pain and reducing the excitation differential of the skin adjacent to the puncture site of the test subjects. It is desirable to mechanically vibrate the skin that surrounds the puncture site. The mechanical vibration also may assist in enhancing the flow of the body fluid sample (*e.g*., the whole blood sample) from the puncture site.

According to one embodiment, the vibration member is a piezoelectric member. A piezoelectric member is desirable because it can economically produce the needed stimulation in a defined area adjacent to the puncture site. The heat from a piezoelectric member, in addition to the vibration, may also assist in enhancing the flow of the body fluid sample (*e.g*., the whole blood sample) from the puncture site.

The piezoelectric member may be powered by a power unit that is integrated into the body portion 12 of the lancing device 10. As shown in FIG. 7, an endcap 314 includes a vibration member 318 (*e.g.*, a piezoelectric member) that forms an aperture 350 in which a lancet is driven therethrough. The vibration member 318 may, for example, be powered by a self-contained battery 360. The battery may be located in the body portion 12 of the lancing device 10. The battery may be a rechargeable battery. The battery 360 may be connected to the vibration member 318 (*e.g.*, a piezoelectric member) by having two plugs on the body portion that receive respective cords 370 that attach to the endcap 314. It is contemplated that other methods for connecting the battery to the vibration member may be employed. It is also contemplated that the amount of power applied to the vibration member may vary and that the test subject may be able to select the desired amount of vibrational energy that is transmitted adjacent to the puncture site.

An example of a piezoelectric member that may be used is manufactured by APC Products, Inc. of Pleasant Gap, P A 16823. It is contemplated that other piezoelectric members may be used from other manufacturers. The piezoelectric member may be a piezoelectric ceramic member.

The piezoelectric member is sized to fit into an opening(s) of the endcap 14 such that the piezoelectric member contacts and mechanically vibrates the skin adj acent to the puncture site. It is desirable that piezoelectric member contacts and mechanically vibrates the skin that surrounds the puncture site. The piezoelectric member may be shaped as a generally circular hollow disk or ring (see FIG. 4) or a generally U-shape (see FIG. 6). It is contemplated, however, that the piezoelectric member may be a variety of other shapes such as a hollow square, rectangle, other polygonal shapes, oval, or other non-polygonal shapes, as well as a variety of other sizes. The piezoelectric member may be a generally circular shape such as shown in the plurality of vibration members 118 in FIG. 5. The thickness of the piezoelectric member may vary, but the thickness should not i nterfere w ith t he p rocess of the lancet drawing the desired amount of body fluid sample. The thickness of the piezoelectric member is generally from about 0.05 to about 0.2 mils.

According to another embodiment, the vibration member may be a rigid member electrically coupled to a DC motor, such as, for example, a coreless DC motor. The rigid member may be, for example, metallic such as brass or polymeric. The rigid member may form an opening to allow a lancet to extend therethrough. The metallic member may be sized and shaped in the same manner as described above with the piezoelectric members. The DC motor is electrically connected to the vibration member via a shaft of the motor. An eccentrically weighted element (*e.g.*, a disk) is connected to the shaft of the DC motor, which in turn is connected or integrated as part of the vibration member. In other words, the eccentric element is attached to the shaft that provides the vibration as the motor turns. This vibrational energy is transferred to the skin tissue adjacent to the puncture site. Depending on the desired amount of vibrational energy, the shaft of the motor may be cycled at different rates such as 13,000 RPM. The DC motor may be powered by a battery such as described above with the piezoelectric members.

According to a further embodiment, the vibration member may be electrically coupled to an outlet using AC current. In this embodiment, the AC current may be converted to DC current using conventional converters known in the art.

The endcap 14 of FIG. 4, for example, is shown as being generally T-shaped. It is contemplated that the endcap may be a variety of other shapes as long as the lancet 16 is capable of extending therethrough to the puncture site and that the endcap is able to detachably connect with the body portion. It is also contemplated that the endcap of the lancing device may be adjustable. One example of an adjustable endcap is disclosed in U.S. Patent No. 6,451,040, which is incorporated by reference in its entirety. These examples of U.S. Patent No. 6,451,040, of course, would need to include a vibrational member such as one of the examples described above. The adjustable endcap may be made to adjust quickly and easily for multiple lancet penetration depths. The endcap may be formed of one or more metallic materials such as stainless steel or made be formed of a non-metal material such as a polymer.

According to one method, the lancing device is adapted to draw body fluid sample (*e.g*., whole blood sample) from the human skin. It is contemplated that the lancing device of the present invention may be used with other skin, such as animal skin. The vibration member of the lancing device is placed in contact with the surface of the skin. The lancet is moved to an extended position such that a tip of the lancet protrudes through the opening formed in the endcap, the vibration member or the combination thereof. The vibration member mechanically vibrates the skin adjacent to a puncture site. The amount of vibration on the skin depends on the amount of energy supplied to the vibrating member and the amount of pressure applied to the vibrating member when contacting the skin of the test subject. A body fluid sample is taken from the skin via the lancet at the puncture site.

According to one process, the vibration member is pressed flush against the test subject's skin surface. During the driving of the lancet, the tip of the lancet according to one embodiment; momentarily protrudes through the opening.

After the lancing of the test subject's skin, a body fluid sample (*e.g.*, a whole blood sample) is produced at the lancet site on the test subject's skin. One analyte of a whole blood sample that may be tested is glucose. It is contemplated, however, that other analytes may be measured such as cholesterol, albumin, fructose, lactate or bilirubin. The present invention is not limited, however, to these specific analytes.

The body fluid sample is then harvested using a biosensor such as an electrochemical- or optic-based biosensor as is known in the art. The biosensor and the device for reading the biosensor can be integrated into the lancing device 10 or be part of a separate testing device according to alternative embodiments of the present invention.

Electrochemical biosensors include a reagent designed to react with the analyte of interest in the body fluid to create an oxidation current at electrodes disposed within the electrochemical biosensor. That current is directly proportional to the concentration of the analyte in the body fluid. Electrochemical biosensors that may be used in connection with various embodiments of the present invention are described in U.S. Patents Nos. 5,120,420 (entitled "Biosensor and a Process for Preparation Thereof"); 5,660,791 ("Fluid Testing Sensor for Use in Dispensing Instrument"); 5,759,364 (entitled "Electrochemical Biosensor"); and 5,798,031 (entitled "Electrochemical Biosensor"); each of which is incorporated herein in its entirety.

Optical biosensors incorporates a reagent designed to produce a colorimetric reaction indicative of the concentration of the analyte of interest in body fluid. The colorimetric reaction is then read by a spectrophotometer incorporated into a testing instrument. Colorimetric testing is described in detail in U.S. Patents Nos. 5,518,689 (entitled "Diffuse Light Reflectance Readhead"); and 5,611,999 (entitled "Diffuse Light Reflectance Readhead"); each of which is incorporated herein by reference in its entirety. An optical biosensor that may be used in connection with various embodiments of the present invention are described in U.S. Patent No. 5,194,393 (entitled: Optical Biosensor and Method of Use), which is incorporated herein by reference in its entirety.

While particular embodiments and applications of the present invention have been illustrated and described, it is to be understood that the invention is not limited to the precise methods disclosed herein and that various modifications, changes, and variations may be apparent from the foregoing descriptions without departing from the spirit and scope of the invention as defined in the appended claims.

## Claims

1. A lancing device for drawing a body fluid sample from the skin, the device comprising:
a body portion;
an endcap detachably connected to the body portion;
a lancet being located within the body portion in a retracted position, the lancet being moveable between a retracted position and an extended position, and adapted to draw body fluid sample from the skin at a puncture site in an extended position; and
a vibration member extending through the endcap and adapted to mechanically vibrate the skin adj acent to the puncture site,
wherein the lancet is adapted to draw the body fluid sample from the skin at a puncture site by extending through an opening formed in the endcap, the vibration member or the combination thereof.

2. The lancing device of claim 1, wherein the opening is formed in the endcap.

3. The lancing device of claim 1, wherein the opening is formed in the vibration member.

4. The lancing device of claim 1, wherein the opening is formed in the combination of the endcap and the vibration member.

5. The lancing device of claim 1, wherein the body portion further includes a pair of latching arms, the pair of latching arms is adapted to detachably connect with the endcap.

6. The lancing device of claim 1, wherein the vibration member is a rigid member.

7. The lancing d evice o f claim 1 further including a plurality of vibration members.

8. A lancing device for drawing a body fluid sample from the skin, the device comprising:
a body portion;
an endcap detachably connected to the body portion;
a lancet being located within the body portion in a retracted position, the lancet being moveable between a retracted position and an extended position, and adapted to draw body fluid sample from the skin at a puncture site in an extended position; and
a piezoelectric member extending through the endcap and adapted to mechanically vibrate the skin adjacent to the puncture site,
wherein the lancet is adapted to draw the body fluid sample from the skin at a puncture site by extending through an opening formed in the endcap, the piezoelectric member or the combination thereof.

9. The lancing device of claim 8, wherein the thickness of the piezoelectric member is from about 0.05 to about 0.2 mils.

10. The lancing device of claim 8, wherein the opening is formed in the endcap.

11. The lancing device of claim 8, wherein the opening is formed in the piezoelectric member.

12. The lancing device of claim 8, wherein the opening is formed in the combination of the endcap and the piezoelectric member.

13. The lancing device of claim 8, wherein the body portion further includes a pair of latching arms, the pair of latching arms is adapted to detachably connect with the endcap.

14. The lancing device of claim 8, wherein the piezoelectric member is a rigid member.

15. The lancing device of claim 8 further including a plurality of piezoelectric members.

16. A method of using a lancing device for drawing a body fluid sample from the skin, the method comprising:
providing a lancing device that comprises a body portion, an endcap, a lancet and a vibration member, the endcap being detachably connected to the body portion, the lancet being located within the body portion in a retracted position, the lancet being moveable between a retracted position and an extended position, and adapted to draw body fluid sample from the skin at a puncture site in an extended position, the vibration member extending through the endcap;
placing the vibration member in contact with the surface of the skin;
moving the lancet to an extended position such that a portion of the lancet protrudes through the opening formed in the endcap, the vibration member or the combination thereof;
mechanically vibrating the vibration member to the skin adjacent to a puncture site; and
taking a body fluid sample from the skin via the lancet at the puncture site.

17. The method of claim 16, wherein the mechanically vibrating of the vibration member surrounds the puncture site.

18. A method of using a lancing device for drawing a body fluid sample from the skin, the method comprising:
providing a lancing device that comprises a body portion, an endcap, a lancet and a piezoelectric member, the endcap being detachably connected to the body portion, the lancet being located within the body portion in a retracted position, the lancet being moveable between a retracted position and an extended position, and adapted to draw body fluid sample from the skin at a puncture site in an extended position, the piezoelectric member extending through the endcap;
placing the piezoelectric member in contact with the surface of the skin;
moving the lancet to an extended position such that a portion of the lancet protrudes through the opening formed in the endcap, the piezoelectric member or the combination thereof;
mechanically vibrating the piezoelectric member to the skin adjacent to a puncture site; and
taking a body fluid sample from the skin via the lancet at the puncture site.

19. The method of claim 18, wherein the moving of the lancet to an extended occurs with a tip of the lancet momentarily protruding through the opening formed in the endcap, the piezoelectric member or the combination thereof.

20. The method of claim 18, wherein the mechanical vibrating of the piezoelectric member surrounds the puncture site.

21. The method of claim 18, wherein the placing of the piezoelectric member in contact with the surface of the skin includes pressing the piezoelectric member flush with the surface of the skin.
